# EUROPÄISCHE PATENTSCHRIFT

(11) **EP 3 016 641 B1**
(45) Veröffentlichungstag und Bekanntmachung des Hinweises auf die Patenterteilung: **05.09.2018**
(21) Anmeldenummer: 14734504.5
(22) Anmeldetag: 03.07.2014
(51) Int. Cl.: A61K 9/70, A61K 45/06, A61K 9/00, A61M 37/00, A61F 13/00, A61F 13/84, A61F 13/02

(54) **TRANSDERMALES THERAPEUTISCHES SYSTEM MIT ELEKTRONISCHEM BAUTEIL**
TRANSDERMAL THERAPEUTIC SYSTEM HAVING AN ELECTRONIC COMPONENT
SYSTÈME THÉRAPEUTIQUE TRANSDERMIQUE DOTÉ D'UN COMPOSANT ÉLECTRONIQUE

(30) Priorität: 03.07.2013 EP 13174880
(43) Veröffentlichungstag der Anmeldung: 11.05.2016
(73) Patentinhaber: LTS Lohmann Therapie-Systeme AG, 56626 Andernach (DE)
(72) Erfinder: LAUX, Wolfgang, 65582 Diez (DE); PLATT, Beatrix, 56745 Hausten (DE); REUM, Nico, 56743 Mendig (DE)
(74) Vertreter: Held, Stephan
(86) Internationale Anmeldenummer: PCT/EP2014/064166
(87) Internationale Veröffentlichungsnummer: WO 2015/001012

(56) Entgegenhaltungen:
- WO-A1-00/44437
- WO-A1-2005/119610
- US-A1- 2004 131 897
- US-A1- 2009 048 556

## Beschreibung

Die Erfindung betrifft transdermale therapeutische Systeme und Verfahren zu deren Herstellung. Insbesondere betrifft die Erfindung transdermale therapeutische Systeme, die mindestens ein elektronisches Bauteil aufweisen.

Das Dokument US 2004/0131897 offenbart transdermale RFID Systeme mit Wirkstoff zum Aufkleben auf die Haut. Unter einem transdermalen therapeutischen System wird in der nachfolgenden Beschreibung eine Vorrichtung zur Verabreichung eines oder mehrerer Wirkstoffe, insbesondere eines oder mehrerer pharmazeutischer Wirkstoffe, über die intakte Haut eines Säugetieres verstanden. Es handelt sich bei transdermalen therapeutischen Systemen um flächige Vorrichtungen, die mindestens einen Wirkstoff enthalten und die auf der Haut beziehungsweise an der Haut eines Säugetiers befestigt werden, vorzugsweise an oder auf der Haut eines Menschen, so dass der in der Vorrichtung enthaltene Wirkstoff über einen längeren Zeitraum hinweg mit konstanter oder zumindest annähernd konstanter Rate an die und über die Haut des Säugetieres abgeben werden kann. Die Befestigung eines transdermalen therapeutischen Systems an oder auf der Haut eines Patienten kann mit Hilfe einer Bandage oder zumindest einem Klebestreifen erfolgen. Bei besonderen Ausführungsformen sind die transdermalen therapeutischen Systeme jedoch haftklebend ausgerüstet. Das bedeutet, dass sie eine haftklebende Fläche aufweisen, mit der sie auf die Haut des Säugetiers geklebt werden können und die einen langanhaltenden Kontakt der Vorrichtung mit der Haut des Säugetieres gewährleistet.

Die Ansprüche offenbaren ein Verfahren zum Herstellen eines transdermalen therapeutischen Systems, das mindestens ein elektronisches Bauteil aufweist, umfassend: - das Beschichten einer Prozessfolie mit einem Haftkleber, - das Aufbringen vorgefertigter elektronischer Bauteile auf die Klebstoffschicht, - das Abdecken der Klebstoffschicht und der darauf aufgebrachten elektronischen Bauteile mit einer Deckfolie, wobei Prozessfolie, Klebstoff und Deckfolie so gewählt sind, dass der Klebstoff stärker an der Deckfolie als an der Prozessfolie haftet und der Klebstofffilm beim Abziehen der Deckfolie an den Kanten der aufgebrachten elektronischen Bauteile reißt, - das Abziehen der Deckfolie, - das Konfektionieren der mit den elektronischen Bauteilen beaufschlagten Prozessfolie zu Rollenware oder das Leporellofalten der mit den elektronischen Bauteilen beaufschlagten Prozessfolie, - das Spenden der elektronischen Bauteile von der mit den elektronischen Bauteilen beaufschlagten Prozessfolie, und - das Überführen der elektronischen Bauteile auf transdermale therapeutische Systeme oder deren unmittelbare Vorstufe. Bei einer Ausführungsform der transdermalen therapeutischen Systeme wird die haftklebende Fläche von einer haftklebenden Polymermatrix gebildet, die auch den Wirkstoff oder mindestens einen der Wirkstoffe enthält. Bei einer zusätzlichen und/oder alternativen Ausführungsform handelt es sich bei der haftklebenden Fläche um eine separate Klebschicht, die auf zumindest einen Bereich der hautseitige Fläche des transdermalen therapeutischen Systems, vorzugsweise auf der hautseitigen Fläche des Wirkstoffreservoirs, aufgebracht ist.

Bei dem mindestens einen Wirkstoffreservoir eines transdermalen therapeutischen Systems handelt es sich entweder um eine Polymermatrix, in der der mindestens eine Wirkstoff enthalten ist, oder um ein beutelförmiges Reservoir, welches von einer Hülle begrenzt wird und eine im Wesentlichen flüssige Wirkstoffzubereitung enthält. Der Begriff "flüssig" umfasst auch dünnflüssige, dickflüssige sowie gelartige Zubereitungen. Die Hülle des beutelförmigen Reservoirs weist zumindest auf seiner der Haut zuzuwendenden Seite eine semipermeable Membran auf, über die der in dem Reservoir enthaltene Wirkstoff abgegeben werden kann und die ggf. eine die Freisetzungsrate des Wirkstoffs kontrollierende Funktion hat. Sofern der mindestens eine Wirkstoff in einer Polymermatrix des transdermalen therapeutischen Systems enthalten ist, ist diese Polymermatrix als Wirkstoffreservoir anzusehen.

Ein transdermales therapeutisches System enthält mindestens einen Wirkstoff, vorzugsweise mindestens einen pharmazeutischen Wirkstoff. Bei dem mindestens einen pharmazeutischen Wirkstoff kann es sich um einen beliebigen, transdermal verabreichbaren pharmazeutischen Wirkstoff handeln. Beispielsweise können Anticholinergika, Parasympatholytika, Antimykotika, MAO-B-Inhibitoren, Serotonin-Antagonisten, Alpha2-Rezeptor-Agonisten, Photosensibilatoren, Hormone und/oder Proteine als pharmazeutische Wirkstoffe eingesetzt werden. In einer Ausführungsform ist der mindestens eine pharmazeutische Wirkstoff aus der Gruppe von Wirkstoffen ausgewählt, die 5-Aminolävulinsäure, Buprenorphin, Capsaicin, Clonidin, Fentanyl, Granisetron, Glyceroltrinitrat, Hydromorphon, Memantine, Oxybutinin, Rivastigmin, Rotigotin, Selegilin und Sertaconazol enthält. Der mindestens eine pharmazeutische Wirkstoff liegt in Form seiner freien Base und/oder mindestens eines seiner pharmazeutisch annehmbaren Salze vor. Der Begriff "pharmazeutisch annehmbares Salz" schließt auch pharmazeutisch annehmbare Säureadditionssalze des Wirkstoffs mit ein. Sofern es sich bei dem mindestens einen Wirkstoff um eine chirale Substanz handelt, liegt der Wirkstoff entweder in Form eines Racemats oder in Form seines pharmazeutisch wirksamen Enantiomers in dem transdermalen therapeutischen System vor.

Bei einer Ausführungsform weisen transdermale therapeutische Systeme eine wirkstoffundurchlässige Rückschicht auf. Bei einer zusätzlichen und/oder alternativen Ausführungsform weisen die transdermalen therapeutischen Systeme eine ablösbare Schutzschicht auf, die die haftklebende Fläche des transdermalen therapeutischen Systems vor dessen Anwendung abdeckt. Die ablösbare Schutzschicht muss vor der Anwendung des transdermalen therapeutischen Systems von dessen haftklebenden Fläche abgezogen werden.

In einem ersten Aspekt betrifft die Erfindung transdermale therapeutische Systeme, die mindestens ein elektronisches Bauteil aufweisen.

In einem zweiten Aspekt betrifft die Erfindung Verfahren zur Herstellung von transdermalen therapeutischen Systemen, die mindestens ein elektronisches Bauteil aufweisen.

Gemäß dem ersten Aspekt betrifft die Erfindung transdermale therapeutische Systeme, die mindestens ein elektronisches Bauteil aufweisen. In einer Ausführungsform ist das mindestens eine elektronische Bauteil ein passives Bauteil, also ein elektronisches Bauteil, das über keine eigene Energieversorgung verfügt.

In einer alternativen Ausführungsform ist das mindestens eine elektronische Bauteil ein aktives Bauteil. Aktive elektronische Bauteile verfügen im Unterschied zu den passiven elektronischen Bauteilen über eine eigene Energieversorgung. In besonderen Ausführungsformen weist das mindestens eine aktive elektronische Bauteil mindestens eine Spannungsquelle auf, die der Energieversorgung des elektronischen Bauteils dient. Bei der mindestens einen Spannungsquelle kann es sich um eine Solarzelle, einen Kondensator oder um ein galvanisches Element handeln, beispielsweise um eine Batterie oder einen Akkumulator.

Gemäß besonderen Ausführungsformen ist das mindestens eine elektronische Bauteil aus der Gruppe von elektronischen Bauteilen ausgewählt, die Sender, Empfänger, Datenspeicher, Sensoren und Messgeräte umfasst.

Bei einer besonderen Ausführungsform ist das mindestens eine elektronische Bauteil ein Funketikett. Das Funketikett kann aus der Gruppe von elektronischen Bauteilen ausgewählt sein, die aus Transpondern, passiven RFID-Transpondern (RFID = radio frequency identification), aktiven RFID-Transpondern, semi-aktiven RFID-Transpondern und semi-passiven RFID-Transpondern besteht. Jeder Transponder umfasst einen Microchip, eine Antenne und einen Träger bzw. ein Gehäuse. Aktive Transponder umfassen zusätzlich die Energiequelle. Der Aufbau eines RFID-Transponders sieht prinzipiell eine Antenne, einen analogen Schaltkreis zum Empfangen und Senden (Transceiver) sowie einen digitalen Schaltkreis und einen permanenten Speicher vor. Der digitale Schaltkreis ist bei komplexeren Modellen ein kleiner Mikrocontroller.

Die RFID-Transponder verfügen über einen mindestens einmal beschreibbaren Speicher, der ihre unveränderliche Identität enthält. Werden mehrfach beschreibbare Speicher eingesetzt, können während der Lebensdauer weitere Informationen abgelegt werden.

Bei besonderen Ausführungsformen ermöglicht das elektronische Bauteil, das mit ihm versehene transdermale therapeutische System zu identifizieren und ggf. zu lokalisieren. Der Transponder bei Funketiketten dient der Speicherung und/oder der Weitergabe von Daten. Beispielsweise können auf einem Transponder gespeicherte Daten an eine Vorrichtung weitergegeben werden, die diese Daten empfangen, verarbeiten, ggf. auch speichern, und anzeigen kann. Bei besonderen Ausführungsformen ermöglicht der Transponder ein Ablegen und/oderAuslesen von Informationen, die der Therapieoptimierung und/oder Therapieüberwachung dienen können. Bei den Informationen, die der Therapieoptimierung und/oder Therapieüberwachung dienen können, kann es sich um Informationen handeln, welcher Wirkstoff in dem transdermalen therapeutischen System enthalten ist und in welcher Dosierung, wann das transdermale therapeutische System appliziert wurde, wann das applizierte transdermale therapeutische System abgenommen werden sollte, wann ein neues transdermales therapeutisches System appliziert werden sollte, ob das transdermale therapeutische System ordnungsgemäß am Patienten befestigt oder abgefallen ist. Bei einer bevorzugten Ausführungsform werden die auf den Applikationszeitpunkt, die Applikationsdauer und/oder das vorgesehene Applikationsende des transdermalen therapeutischen Systems mittels Aktivierung des Funketiketts durch den Kontakt des transdermalen therapeutischen Systems mit der Haut generiert.

Das elektronische Bauteil kann in Größe und Form variieren. Bei einer Ausführungsform liegt das elektronische Bauteil in Form eines nicht flexiblen Elements vor, welches eine Dicke von zwischen etwa 10 µm bis etwa 1,5 mm aufweist.

Bei einer Ausführungsform ist das mindestens eine elektronische Bauteil auf der Rückschicht des transdermalen therapeutischen Systems aufgebracht. Diese Anordnung bietet den Vorteil, dass bereits vorgefertigte transdermale therapeutische Systeme mit einem elektronischen Bauteil versehen werden können.

Bei einer anderen Ausführungsform ist das mindestens eine elektronische Bauteil in das transdermale therapeutische System integriert. Das bedeutet, dass das mindestens eine elektronische Bauteil beispielsweise in einer wirkstoffhaltigen Polymermatrix eingebettet ist. Bei einer zusätzlichen und/oder alternativen Ausführungsform ist das mindestens eine elektronische Bauteil zwischen zwei Matrixschichten oder zwischen dem Wirkstoffreservoir und der wirkstoffundurchlässigen Rückschicht angeordnet. Diese Ausführungsformen haben den Vorteil, dass das elektronische Bauteil zu einem integralen Bestandteil des transdermalen therapeutischen Systems wird und es nicht möglich ist, das elektronische Bauteil zu entfernen, ohne dass transdermale therapeutische System zu zerstören.

Gemäß dem zweiten Aspekt betrifft die Erfindung Verfahren zur Herstellung von transdermalen therapeutischen Systemen, die mindestens ein elektronisches Bauteil, vorzugsweise ein Funketikett aufweisen.

Bei den Verfahren nach dem zweiten Aspekt der Erfindung werden die elektronischen Bauteile separat hergestellt, und die transdermalen therapeutischen Systeme werden entweder während oder nach ihrer Herstellung mit mindestens einem der vorgefertigten elektronischen Bauteile versehen. Das bedeutet bei einer ersten Ausführungsform, dass mindestens ein vorgefertigtes elektronisches Bauteil auf einem vorgefertigten transdermalen therapeutischen System befestigt wird. In einer zusätzlichen und/oder alternativen Ausführungsform wird mindestens ein vorgefertigtes elektronisches Bauteil auf einem noch nicht vollständig vorgefertigten transdermalen therapeutischen System befestigt. In noch einer zusätzlichen und/oder alternativen Ausführungsform wird mindestens ein vorgefertigtes elektronisches Bauteil während der Herstellung des transdermalen therapeutischen Systems in selbiges integriert.

Bei der erstgenannten Ausführungsform wird mindestens ein separat hergestelltes elektronisches Bauteil auf der Rückschicht eines vorgefertigten transdermalen therapeutischen Systems oder seiner unmittelbaren Vorstufe befestigt. Hierzu wird bei einer Variante dieser Ausführungsform eine Prozessfolie zunächst vollflächig mit einem Haftklebstoff beschichtet. In einem weiteren Schritt werden die elektronischen Bauteile auf die Klebschicht aufgelegt und anschließend mit einer Deckfolie abgedeckt. Anschließend wird die Deckfolie wieder abgezogen, wobei der Haftklebstoff in den Bereichen, in denen keine elektronischen Bauteile aufliegen, mit dem Abziehen der Deckfolie von der Prozessfolie entfernt wird. Die mit den elektronischen Bauteilen beaufschlagte Prozessfolie wird als Rollenware konfektioniert oder in einer anderen Ausführungsform einer Leporellofaltung unterzogen. In einem weiteren Verfahrensschritt werden die einzelnen elektronischen Bauteile einschließlich der an ihnen haftenden Klebstoffschicht mittels einer Etikettiermaschine auf transdermale therapeutische Systeme oder deren unmittelbare Vorstufe überführt.

Unter vorgefertigten transdermalen therapeutischen Systemen werden bereits vereinzelte, gebrauchsfertige transdermale therapeutische Systeme verstanden, also transdermale therapeutische Systeme die bereits ihre vorgesehene Fläche haben. Mit der unmittelbaren Vorstufe von transdermalen therapeutischen Systemen wird das Laminat aus wirkstoffundurchlässiger Rückschicht, wirkstoffhaltigem Reservoir und ablösbarer Schutzschicht verstanden, aus dem die einzelnen transdermalen therapeutischen Systeme durch Schneiden oder Stanzen vereinzelt werden.

Das Verfahren gemäß der ersten Ausführungsform umfasst somit:
- das Beschichten einer Prozessfolie mit einem Haftkleber,
- das Aufbringen vorgefertigter elektronischer Bauteile auf die Klebstoffschicht,
- das Abdecken oder Kaschieren der Klebstoffschicht und der darauf aufgebrachten elektronischen Bauteile mit einer Deckfolie, wobei Prozessfolie, Klebstoff und Deckfolie so gewählt sind, dass der Klebstoff stärker an der Deckfolie als an der Prozessfolie haftet und der Klebstofffilm beim Abziehen der Deckfolie an den Kanten der aufgebrachten elektronischen Bauteile reißt, das Abziehen der Deckfolie,
- das Konfektionieren der mit den elektronischen Bauteilen beaufschlagten Prozessfolie zu Rollenware oder das Leporellofalten der mit den elektronischen Bauteilen beaufschlagten Prozessfolie,
- das Spenden der elektronischen Bauteile von der mit den elektronischen Bauteilen beaufschlagten Prozessfolie mittels Etikettiermaschine, und
- das Überführen der elektronischen Bauteile auf transdermale therapeutische Systeme oder deren unmittelbare Vorstufe.

Die Prozessfolie weist zumindest eine für den Klebstoff, mit dem die Prozessfolie beschichtet werden soll, dehäsive Oberfläche auf. Für Silikonhaftkleber werden vorzugsweise perfluorierte Prozessfolien verwendet. Bevorzugte Prozessfolien für Silikonhaftkleber sind beispielsweise die am Anmeldetag der vorliegenden Offenbarung unter dem Handelsnamen Scotchpak™ von der Firma 3M, St. Paul, MN, kommerziell erhältlichen Polyesterfolien. Zu den besonders bevorzugten perfluorierten Prozessfolien gehören beispielsweise die unter den Handelsbezeichnungen Scotchpak™ 1022 und Scotchpak™ 9755 vertriebenen Polyesterfolien, die mit Fluorpolymer beschichtet sind, so dass gemäß den Herstellerangaben ein "Liner Release" von < 1,0 N/25,4 mm (bei Scotchpak™ 1022) beziehungsweise von <0,4 N/25,4 mm (bei Scotchpak™ 9755) resultiert. Bevorzugte Prozessfolien, die mit einem hydrophilen Haftkleber beschichtet werden sollen, beispielsweise einem hydrophilen Acrylathaftkleber oder einem Polyisobutylen, weisen dagegen eine silikonisierte Oberfläche auf.

Eine für hydrophile Haftkleber geeignete Prozessfolie ist beispielsweise silikonisiertes Papier.

Die Prozessfolie wird mit einem Haftklebstoff beschichtet. Die Beschichtung erfolgt vorzugsweise vollflächig. Die Beschichtung der Prozessfolie mit dem Haftklebstoff erfolgt derart, dass ein Klebstofffilm mit einer im Wesentlichen gleichmäßigen Dicke erzeugt wird. Die Dicke des Klebstofffilms beträgt mindestens etwa 10 µm, vorzugsweise etwa 30 µm. Die Dicke des Klebstofffilms sollte jedoch nicht mehr als etwa 500 µm betragen, vorzugsweise eine Dicke von etwa 200 µm nicht überschreiten. Ein Klebstofffilm dieser Dicke ermöglicht ein sicheres und genaues Positionieren der elektronischen Bauteile, ohne dass es zu einer unerwünscht großen Lateralbewegung der auf der Prozessfolie aufgebrachten elektronischen Bauteile kommt, sowie ein verlässliches Reißen des Klebstofffilms an den Rändern der elektronischen Bauteile, wenn die Deckfolie abgezogen wird.

Bei den elektronischen Bauteilen handelt es sich vorzugsweise um die vorgenannten Funketiketten/Transponder.

Bei der Deckfolie kann es sich um einen beliebigen Polymerfilm handeln, an den der Haftkleber haftet. Geeignete Deckfolien bestehen beispielsweise aus einem Polyester wie z.B. Polyethylenterephthalat. Die Deckfolie muss flexibel sein, damit sie beim Abziehen über eine Umlenkrolle oder Kante gezogen werden kann. Vorzugsweise wird die Deckfolie unter Bildung eines spitzen Winkels abgezogen.

Bei dem Verfahren sind Prozessfolie, Klebstoff und Deckfolie so zu wählen, dass der Klebstoff stärker an der Deckfolie als an der Prozessfolie haftet und der Klebstofffilm beim Abziehen der Deckfolie an den Kanten der aufgebrachten elektronischen Bauteile reißt.

Beim Abdecken der Klebstoffschicht und der auf der Klebstoffschicht aufgebrachten elektronischen Bauteile mit der Deckfolie haftet der Klebstoff in den Bereichen an der Deckfolie, in denen er nicht von den elektronischen Bauteilen abgedeckt wird. Beim nachfolgenden Abziehen der Deckfolie wird der an ihr haftenden Klebstoffilm in den Bereichen, in denen er nicht von elektronischen Bauteilen abgedeckt wird, von der Prozessfolie abgezogen. Dabei reißt der Klebstofffilm an den Kanten der auf den Klebstofffilm aufgebrachten elektronischen Bauteile, so dass die elektronischen Bauteile nicht mit abgezogen werden, sondern einschließlich der von ihnen abgedeckten Bereiche des Klebstofffilms auf der Prozessfolie verbleiben. Auf diese Weise wird eine mit elektronischen Bauteilen beaufschlagte Prozessfolie erhalten, die im Wesentlichen keine freien Klebstoffflächen aufweist, die das weitere Verwenden der dann zu Rollen oder Stapeln konfektionierten Prozessfolie beeinträchtigen könnte.

Die mit elektronischen Bauteilen beaufschlagte Prozessfolie wird zu Rollenware konfektioniert oder mittels Leporellofaltung zu einem Stapel geformt. Dadurch kann die mit elektronischen Bauteilen beaufschlagte Prozessfolie einer Etikettiermaschine zugeführt werden, mit deren Hilfe die mit dem Klebstofffilm bewährten elektronischen Bauteile in einem automatisierten Verfahrensschritt auf transdermale therapeutische Systeme oder deren unmittelbare Vorstufe transferiert werden können.

Der Transfer der klebstoffbewährten elektronischen Bauteile von der Prozessfolie auf transdermale therapeutische Systeme oder deren unmittelbare Vorstufe kann manuell oder maschinell erfolgen. Der maschinelle Transfer kann wie vorstehend mit Hilfe einer Etikettiermaschine erfolgen. Bei einer andere Vorgehensweise können die einzelnen elektronischen Bauteile von einem Roboterarm gegriffen, von der Prozessfolie abgenommen und auf die transdermalen therapeutischen Systeme oder deren unmittelbare Vorstufe positioniert werden.

Grundsätzlich ist es möglich, die elektronischen Bauteile auf bereits fertige, d.h. auf schon vereinzelte transdermale therapeutische Systeme zu übertragen. Bei einer anderen Ausführungsform werden die einzelnen elektronischen Bauteile auf die unmittelbare Vorstufe der transdermalen therapeutischen Systeme übertragen, also auf ein Laminat, das eine wirkstoffundurchlässige Rückschicht, mindestens ein wirkstoffhaltiges Reservoir und optional bereits eine ablösbare Schutzschicht umfasst. Nach dem Übertragen der elektronischen Bauteile auf das Laminat werden die individuellen transdermalen therapeutischen Systeme so vereinzelt, dass sie mindestens eines der elektronischen Bauteile aufweisen. Das Vereinzeln der transdermalen therapeutischen Systeme erfolgt beispielsweise indem die einzelnen transdermalen therapeutischen Systeme aus dem Laminat ausgestanzt oder ausgeschnitten werden.

Bei einer anderen Ausgestaltung der ersten Ausführungsform werden nicht mit Klebstoff bewährte elektronischen Bauteile auf transdermale therapeutische Systeme oder deren unmittelbare Vorstufe übertragen. Bei dieser Ausgestaltung wird mindestens eine Klebstofffläche je transdermalem therapeutischen System mit Hilfe von Siebdruck auf die Rückschicht des transdermalen therapeutischen Systems oder dessen unmittelbaren Vorläufer aufgebracht. Bei diesem Vorgehen werden Klebstoffflächen im Wesentlichen an den Positionen der transdermalen therapeutischen Systeme oder ihrer unmittelbaren Vorstufe angebracht, an denen elektronischen Bauteile befestigt werden sollen. Die aufgebrachten Klebstoffflächen haben im Wesentlichen die gleiche Fläche und Form wie die zu befestigenden elektronischen Bauteile.

Bei dieser Ausgestaltung müssen die zu übertragenden elektronischen Bauteile nicht mit einem Haftklebstoff bewährt sein, weil der für das Befestigen der elektronischen Bauteile erforderliche Kleber auf die wirkstoffundurchlässige Rückschicht aufgebracht wird. Auch bei dieser Ausgestaltung können die elektronischen Bauteile manuell oder maschinell auf die transdermalen therapeutischen Systeme oder ihrer unmittelbaren Vorstufe transferiert werden. Bei einer Variante des maschinellen Transfers werden beispielsweise in einem Rohr gestapelte elektronische Bauteile von unten her mit Hilfe eines mit einem Vakuumsaugnapf versehenen Arms von dem Spender auf transdermale therapeutische Systeme transferiert. Bei einer anderen Variante werden die einzelnen elektronischen Bauteile von einem Roboterarm gegriffen, vorzugsweise seitlich gegriffen, und auf eine Klebstofffläche auf der Rückschicht eines transdermalen therapeutischen Systems oder seiner unmittelbare Vorstufe positioniert.

In einer anderen Ausführungsform wird mindestens ein elektronisches Bauteil in ein transdermales therapeutisches System integriert. Das bedeutet, dass das mindestens eine elektronischen Bauteile zwischen zwei Schichten eines mehrschichtigen transdermalen therapeutischen Systems angeordnet wird, beispielsweise zwischen zwei wirkstoffhaltigen Schichten oder zwischen dem wirkstoffhaltigen Reservoir und der wirkstoffundurchlässigen Rückschicht. Alternativ oder zusätzlich kann mindestens ein elektronisches Bauteil in eine Polymerschicht des transdermalen therapeutischen Systems eingebettet werden.

Bei einer Ausgestaltung dieser Ausführungsform werden vorgefertigte elektronische Bauteile nicht auf bereits fertig gestellte transdermale therapeutische Systeme übertragen, sondern während der Herstellung der transdermalen therapeutischen Systeme in diese integriert, beispielsweise indem die elektronischen Bauteile auf die zuletzt hergestellte Schicht eines Laminats aufgebracht und anschließend mit einer weiteren Schicht abgedeckt werd. Beispielsweise werden die elektronischen Bauteile direkt auf eine wirkstoffhaltige Polymerschicht aufgelegt, die im fertigen transdermalen therapeutischen System die wirkstoffhaltige Polymermatrix oder einen Teil der wirkstoffhaltigen Polymermatrix bildet, und von einer weiteren wirkstoffhaltigen Polymerschicht, wirkstofffreien Polymerschicht oder wirkstoffundurchlässigen Rückschicht abgedeckt. Sofern es sich bei der Schicht, auf die die elektronischen Bauteile aufgelegt werden, um eine haftklebende Schicht handelt, müssen die elektronischen Bauteile nicht mit einer haftklebenden Fläche versehen sein. Sofern es sich bei der Schicht, auf die die elektronischen Bauteile aufgelegt werden, nicht um eine haftklebende Schicht handelt, können die elektronischen Bauteile mit einer haftklebenden Fläche versehen sein, beispielsweise wie in dem erstgenannten Ausführungsbeispiel. In einem späteren Verfahrensschritt wird mindestens eine weitere Schicht, beispielsweise mindestens eine weitere wirkstoffhaltige Polymerschicht und/oder eine wirkstoffundurchlässige Rückschicht, auf die mit elektronischen Bauteilen versehene Schicht aufgebracht und die individuellen transdermalen therapeutischen Systeme aus dem resultierenden Laminat vereinzelt, so dass jedes einzelne transdermale therapeutische System zumindest ein elektronisches Bauteile aufweist.

Diese Ausführungsform hat den Vorteil, dass das mindestens eine elektronischen Bauteile zwischen wirkstoffhaltiger Polymermatrix und wirkstoffundurchlässiger Rückschicht angeordnet ist und deshalb nicht von dem transdermalen therapeutischen System entfernt werden kann, ohne es zu zerstören.

In einer weiteren Ausgestaltung dieser Ausführungsform wird mindestens ein elektronisches Bauteil in eine Polymermatrix eingebettet. Hierbei kann das elektronische Bauteil gleichsam in eine Polymermatrix eingegossen oder eingepresst werden, bevor eine weitere Schicht, beispielsweise eine weitere Matrixschicht oder die wirkstoffundurchlässige Rückschicht, auf die Polymermatrix aufgebracht wird.

Eine Ausführungsform des erfindungsgemäßen Verfahrens wird nachfolgend unter Bezug auf die Figuren näher erläutert. Dabei ist zu berücksichtigen, dass die Figuren lediglich veranschaulichende Bedeutung haben und die Erfindung in keiner Weise einschränken.

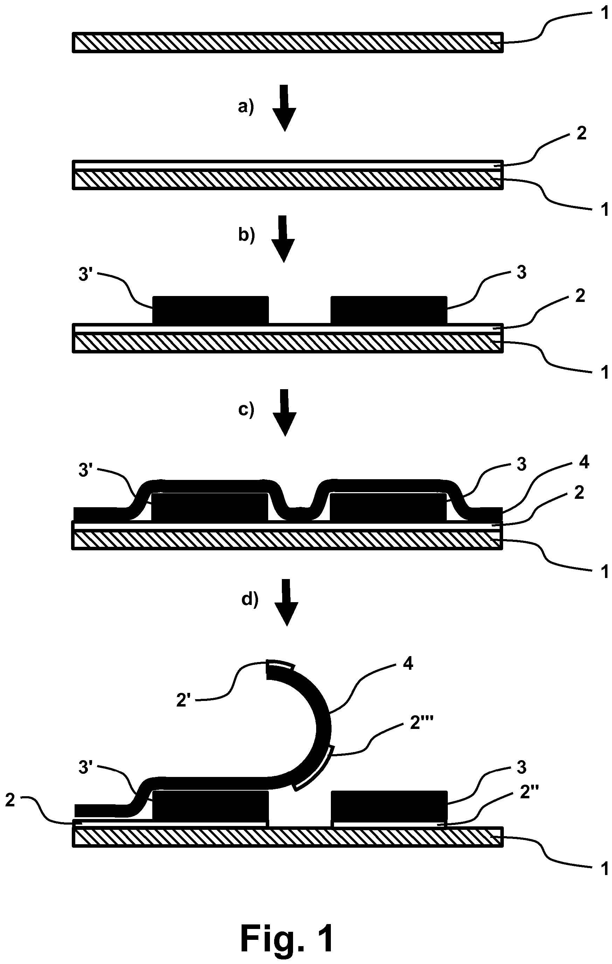

Fig. 1 ist eine schematische Veranschaulichung einiger Verfahrensschritte bei einer Ausführungsform des Verfahrens zur Herstellung transdermaler therapeutischer Systeme mit einem elektronischen Etikett.

Zunächst wurde eine Bahn silikonisiertes Papier als Prozessfolie 1 bereitgestellt. Die Prozessfolie 1 wurde in einem Verfahrensschritt a) vollflächig mit einer Klebstoffschicht 2 beschichtet. Bei dem Klebstoff handelte es sich um Poly[(2-ethylhexyl)acrylat-co-methylacrylat-co-acrylsäure-co-(2,3-epoxypropyl)methacrylat] (61,5:33:5,5:0,02). Dieser Acrylathaftkleber ist im Handel unter der Warenbezeichnung DuroTak® 2353 von der Firma National Starch, jetzt Henkel, erhältlich. Die Dicke der Klebstoffschicht 2 auf der Prozessfolien 1 betrug 30 µm. Anschließend wurden Funketiketten 3, 3' auf die Klebstoffschicht 2 platziert (Verfahrensschritt b)). In einem sich daran anschließenden Verfahrensschritt (Schritt c)) wurden die Funketiketten 3, 3' sowie die noch freie Fläche der Klebstoffschicht 2 mit einer Polyethylenterephthalatfolie als Deckfolie 4 abgedeckt. In den Bereichen, in denen die Deckfolie 4 in Kontakt mit der Klebstoffschicht 2 kam, haftete die Deckfolie 4 an der Klebstoffschicht 2. Sodann wurde in Schritt d) die Deckfolie 4 wieder abgezogen. Dabei hafteten die Bereiche 2', 2'" der Klebstoffschicht 2, die mit der Deckfolie 4 in Kontakt standen an der Deckfolie 4 und wurden zusammen mit der Deckfolie 4 abgezogen. Die von den Funketiketten 3, 3' bedeckten Bereiche 2" der Klebstoffschicht 2 hafteten nicht an der Deckfolie 4. Beim Abziehen der Deckfolie 4 wurden die an der Deckfolie 4 haftenden Bereiche der Klebstoffschicht 2 von den Bereichen der Klebstoffschicht getrennt, die von den Funketiketten 3, 3' abgedeckt waren. Auf diese Weise verblieben die Funketiketten 3, 3' auf einer zu ihrer Grundfläche deckungsgleichen Klebstoffschicht auf der Prozessfolie 1.

## Patentansprüche

1. Ein Verfahren zum Herstellen eines transdermalen therapeutischen Systems, das mindestens ein elektronisches Bauteil aufweist, umfassend
- das Beschichten einer Prozessfolie mit einem Haftkleber,
- das Aufbringen vorgefertigter elektronischer Bauteile auf die Klebstoffschicht,
- das Abdecken der Klebstoffschicht und der darauf aufgebrachten elektronischen Bauteile mit einer Deckfolie, wobei Prozessfolie, Klebstoff und Deckfolie so gewählt sind, dass der Klebstoff stärker an der Deckfolie als an der Prozessfolie haftet und der Klebstofffilm beim Abziehen der Deckfolie an den Kanten der aufgebrachten elektronischen Bauteile reißt,
- das Abziehen der Deckfolie,
- das Konfektionieren der mit den elektronischen Bauteilen beaufschlagten Prozessfolie zu Rollenware oder das Leporellofalten der mit den elektronischen Bauteilen beaufschlagten Prozessfolie,
- das Spenden der elektronischen Bauteile von der mit den elektronischen Bauteilen beaufschlagten Prozessfolie, und
- das Überführen der elektronischen Bauteile auf transdermale therapeutische Systeme oder deren unmittelbare Vorstufe.

2. Das Verfahren gemäß Anspruch 1, **dadurch gekennzeichnet, dass** es sich bei der Prozessfolie um eine perfluorierte Prozessfolie und bei dem Haftkleber um einen Silikonhaftkleber handelt.

3. Das Verfahren gemäß Anspruch 1, **dadurch gekennzeichnet, dass** die Prozessfolie eine silikonisierte Oberfläche aufweist und der Haftkleber ein hydrophiler Haftkleber ist, vorzugsweise ein hydrophiler Acrylathaftkleber oder ein Polyisobutylen.

4. Das Verfahren gemäß einem der vorhergehenden Ansprüche, **dadurch gekennzeichnet, dass** die Beschichtung mit dem Haftklebstoff derart erfolgt, dass ein Klebstofffilm mit einer im Wesentlichen gleichmäßigen Dicke erzeugt wird.

5. Das Verfahren gemäß Anspruch 4, **dadurch gekennzeichnet, dass** die Dicke des Klebstofffilms mindestens 10 µm beträgt, vorzugsweise etwa 30 µm.

6. Das Verfahren gemäß Anspruch 4 oder 5, **dadurch gekennzeichnet, dass** die Dicke des Klebstofffilms nicht mehr als 500 µm beträgt, vorzugsweise nicht mehr als 200 µm.

7. Das Verfahren gemäß einem der vorhergehenden Ansprüche, **dadurch gekennzeichnet, dass** das elektronische Bauteil ein Funketikett ist, das über keine eigene Energieversorgung verfügt.

8. Das Verfahren gemäß einem der Ansprüche 1 bis 6, **dadurch gekennzeichnet, dass** das elektronische Bauteil ein Funketikett ist, das über eine eigene Energieversorgung verfügt.

9. Das Verfahren gemäß Anspruch 8, **dadurch gekennzeichnet, dass** das Funketikett eine Spannungsquelle aufweist, die vorzugsweise aus der Gruppe ausgewählt ist, die Kondensatoren, Solarzellen und galvanische Elemente umfasst.

10. Das Verfahren gemäß einem der Ansprüche 7 bis 9, **dadurch gekennzeichnet, dass** das Funketikett aus der Gruppe ausgewählt ist, die aus Transpondern, passiven RFID-Transpondern (RFID = radio frequency identification), aktiven RFID-Transpondern, semi-aktiven RFID-Transpondern und semi-passiven RFID-Transpondern besteht.

11. Das Verfahren gemäß einem der vorhergehenden Ansprüche, **dadurch gekennzeichnet, dass** die Deckfolie aus einem Polyester besteht, vorzugsweise aus Polyethylenterepthalat.

12. Das Verfahren gemäß einem der vorhergehenden Ansprüche, **dadurch gekennzeichnet, dass** die Deckfolie beim Abziehen über eine Umlenkrolle oder Kante gezogen wird, vorzugsweise unter Bildung eines spitzen Winkels.

13. Das Verfahren gemäß einem der vorhergehenden Ansprüche, **dadurch gekennzeichnet, dass** das Spenden der elektronischen Bauteile mittels einer Etikettiermaschine erfolgt.

14. Das Verfahren gemäß einem der vorhergehenden Ansprüche, **dadurch gekennzeichnet, dass** das transdermale therapeutische System oder seine unmittelbare Vorstufe mindestens einen Wirkstoff enthält, der vorzugsweise aus der Gruppe von Wirkstoffen ausgewählt ist, die Anticholinergika, Parasympatholytika, Antimykotika, MAO-B-Inhibitoren, Serotonin-Antagonisten, Alpha2-Rezeptor-Agonisten, Photo- sensibilisatoren, Hormonen und Proteine umfasst, vorzugsweise aus der Gruppe von Wirkstoffen, die 5-Aminolävulinsäure, Buprenorphin, Capsaicin, Clonidin, Fentanyl, Granisetron, Glyceroltrinitrat, Hydromorphon, Memantine, Oxybutinin, Rivastigmin, Rotigotin, Selegilin und Sertaconazol umfasst.

## Claims

1. A method for producing a transdermal therapeutic system which has at least one electronic component, comprising
- coating a process film with a pressure-sensitive adhesive,
- applying prefabricated electronic components to the adhesive layer,
- covering the adhesive layer and the electronic components applied thereto with a covering film, wherein the process film, adhesive and covering film are selected such that the adhesive adheres more strongly to the covering film than to the process film and the adhesive film when the covering film is pulled off tears at the edges of the applied electronic components,
- pulling off the covering film,
- finally forming the process film which is supplied with the electronic components into rolls, or concertina-folding of the process film which is supplied with the electronic components,
- dispensing the electronic components from the process film which is supplied with the electronic components, and
- transferring the electronic components to transdermal therapeutic systems or the immediate precursor thereof.

2. The method according to Claim 1, **characterised in that** the process film is a perfluorinated process film and the pressure-sensitive adhesive is a silicone pressure-sensitive adhesive.

3. The method according to Claim 1, **characterised in that** the process film has a siliconised surface and the pressure-sensitive adhesive is a hydrophilic pressure-sensitive adhesive, preferably a hydrophilic acrylate pressure-sensitive adhesive or a polyisobutylene.

4. The method according to one of the preceding claims, **characterised in that** the coating with the pressure-sensitive adhesive takes place such that an adhesive film with a substantially uniform thickness is produced.

5. The method according to Claim 4, **characterised in that** the thickness of the adhesive film is at least 10 µm, preferably approximately 30 µm.

6. The method according to Claim 4 or Claim 5, **characterised in that** the thickness of the adhesive film is no more than 500 µm, preferably no more than 200 µm.

7. The method according to one of the preceding claims, **characterised in that** the electronic component is a radio tag which does not have its own power supply.

8. The method according to one of Claims 1 to 6, **characterised in that** the electronic component is a radio tag which has its own power supply.

9. The method according to Claim 8, **characterised in that** the radio tag has a voltage source which is preferably selected from the group comprising capacitors, solar cells and galvanic elements.

10. The method according to one of Claims 7 to 9, **characterised in that** the radio tag is selected from the group consisting of transponders, passive RFID transponders (RFID = radio frequency identification), active RFID transponders, semi-active RFID transponders and semi-passive RFID transponders.

11. The method according to one of the preceding claims, **characterised in that** the covering film consists of a polyester, preferably of polyethylene terephthalate.

12. The method according to one of the preceding claims, **characterised in that** the covering film when being pulled off is drawn over a deflecting roll or edge, preferably forming an acute angle.

13. The method according to one of the preceding claims, **characterised in that** the dispensing of the electronic components takes place by means of a labelling machine.

14. The method according to one of the preceding claims, **characterised in that** the transdermal therapeutic system or its immediate precursor contains at least one active substance which is preferably selected from the group of active substances which comprises anticholinergics, parasympatholytics, antimycotics, MAO-B inhibitors, serotonin antagonists, alpha2-receptor agonists, photosensitisers, hormones and proteins, preferably from the group of active substances which comprises 5-aminolevulinic acid, buprenorphine, capsaicin, clonidine, fentanyl, granisetron, glycerol trinitrate, hydromorphone, memantine, oxybutynin, rivastigmine, rotigotine, selegiline and sertaconazole.

## Revendications

1. Procédé de fabrication d'un système thérapeutique transdermique, doté d'au moins un composant électronique, comprenant :
- le revêtement d'un film de processus avec un adhésif sensible à la pression,
- l'application de composants électroniques préfabriqués sur la couche adhésive,
- le recouvrement de la couche adhésive et des composants électroniques déposés sur celle-ci avec un film de recouvrement, le film de processus, l'adhésif et le film de recouvrement étant choisis de manière que l'adhésif adhère plus fortement au film de recouvrement qu'au film de de processus et que le film adhésif se déchire sur les bords des composants électroniques appliqués lors du retrait du film de recouvrement,
- le retrait du film de recouvrement,
- la confection du film de processus sur lequel ont été appliqués les composants électroniques en un rouleau ou le pliage Leporello du film de processus sur lequel ont été appliqués les composants électroniques,
- la distribution des composants électroniques du film de processus sur lequel ont été appliqués les composants électroniques, et
- le transfert des composants électroniques sur des systèmes thérapeutiques transdermiques ou leurs précurseurs immédiats.

2. Procédé selon la revendication 1, **caractérisé en ce que** le film de processus est un film de processus perfluoré et l'adhésif sensible à la pression est un adhésif sensible à la pression siliconé.

3. Procédé selon la revendication 1, **caractérisé en ce que** le film de processus présente une surface siliconée et l'adhésif sensible à la pression est un adhésif sensible à la pression hydrophile, de préférence un adhésif sensible à la pression d'acrylate hydrophile ou un polyisobutylène.

4. Procédé selon l'une des revendications précédentes, **caractérisé en ce que** le revêtement avec l'adhésif sensible à la pression s'effectue de sorte qu'un film adhésif d'une épaisseur sensiblement régulière soit produit.

5. Procédé selon la revendication 4, **caractérisé en ce que** l'épaisseur du film adhésif est d'au moins 10 µm, de préférence d'environ 30 µm.

6. Procédé selon la revendication 4 ou 5, **caractérisé en ce que** l'épaisseur du film adhésif n'est pas supérieure à 500 µm, de préférence non supérieure à 200 µm.

7. Procédé selon l'une des revendications précédentes, **caractérisé en ce que** le composant électronique est une radio-étiquette qui ne dispose pas d'une alimentation en énergie autonome.

8. Procédé selon l'une des revendications 1 à 6, **caractérisé en ce que** le composant électronique est une radio-étiquette qui dispose d'une alimentation en énergie autonome.

9. Procédé selon la revendication 8, **caractérisé en ce que** la radio-étiquette présente une source de tension qui est choisie de préférence dans le groupe comprenant les condensateurs, les cellules photovoltaïques et les piles électrochimiques.

10. Procédé selon l'une des revendications 7 à 9, **caractérisé en ce que** la radio-étiquette est choisie dans le groupe constitué des transpondeurs, des transpondeurs passifs RFID (RFID = identification par radiofréquence), des transpondeurs actifs RFID, des transpondeurs semi-actifs RFID et des transpondeurs semi-passifs RFID.

11. Procédé selon l'une des revendications précédentes, **caractérisé en ce que** le film de recouvrement est constitué d'un polyester, de préférence de polyéthylène téréphtalate.

12. Procédé selon l'une des revendications précédentes, **caractérisé en ce que** le film de recouvrement est tiré par le biais d'un rouleau de déviation ou d'une bordure lors du retrait, de préférence en formant un angle aigu.

13. Procédé selon l'une des revendications précédentes, **caractérisé en ce que** la distribution des composants électroniques s'effectue au moyen d'une machine d'étiquetage.

14. Procédé selon l'une des revendications précédentes, **caractérisé en ce que** le système thérapeutique transdermique ou son précurseur immédiat contient au moins une substance active, qui est choisie de préférence dans le groupe des substances actives qui comprend les anti-cholinergiques, les parasympatholytiques, les antimycosiques, les inhibiteurs de la MAO-B, les antagonistes de la sérotonine, les agonistes du récepteur alpha-2, les photosensibilisants, les hormones et les protéines, de préférence dans le groupe des substances actives qui comprend l'acide 5-aminolévulinique, la buprénorphine, la capsaïcine, la clonidine, le fentanyle, le granisétron, le trinitrate de glycérol, l'hydromorphone, la mémantine, l'oxybutinine, la rivastigmine, la rotigotine, la sélégiline et le sertaconazol.
